# EUROPEAN PATENT SPECIFICATION

(11) **EP 3 511 294 B1**
(45) Date of publication and mention of the grant of the patent: **15.06.2022**
(21) Application number: 17848943.1
(22) Date of filing: 11.05.2017
(51) Int. Cl.: C01B 3/00, C01B 3/22, C07D 401/06, C07D 213/00

(54) **METHOD FOR STORING AND DISCHARGING HYDROGEN USING PYRIDINE-BASED HYDROGEN STORAGE MATERIAL**
VERFAHREN ZUR SPEICHERUNG UND AUSGABE VON WASSERSTOFF MITTELS EINES WASSERSTOFFSPEICHERSTOFFES AUF PYRIDINBASIS
PROCÉDÉ DE STOCKAGE ET DE DÉCHARGE D'HYDROGÈNE À L'AIDE D'UN MATÉRIAU DE STOCKAGE D'HYDROGÈNE À BASE DE PYRIDINE

(30) Priority: 09.09.2016 KR 20160116140
(43) Date of publication of application: 17.07.2019
(73) Proprietor: Korea Research Institute of Chemical Technology, Daejeon 34114 (KR); IUCF-HYU (Industry-University Cooperation Foundation Hanyang University), Seoul 04763 (KR); Postech Academy-Industry Foundation, Pohang-si, Gyeongsangbuk-do 37673 (KR)
(72) Inventor: PARK, Ji Hoon, Pyeongtaek-si Gyeonggi-do 17891 (KR); KIM, Soo Min, Daejeon 34119 (KR); CHANG, Tae Sun, Daejeon 34140 (KR); KIM, Beon Sik, Daejeon 34140 (KR); JEONG, Kwan Yong, Daejeon 34116 (KR); SUH, Young-Woong, Seoul 06069 (KR); OH, Jinho, Seoul 05019 (KR); KIM, Tae Wan, Incheon 21949 (KR); HAN, Jeong Woo, Gwacheon-si Gyeonggi-do 13835 (KR); KWON, Hyunguk, Seoul 08275 (KR); LEE, Myeong Jung, Goesan-gun Chungcheongbuk-do 28017 (KR)
(74) Representative: Epping - Hermann - Fischer
(86) International application number: PCT/KR2017/004853
(87) International publication number: WO 2018/048058

(56) References cited:
- CN-A- 101 823 692
- CN-A- 104 555 914
- DE-A1-102014 210 464
- JP-A- 2009 242 232
- KR-A- 20010 102 934
- KR-A- 20060 022 651
- AGAI, B. et al.: "Convenient, Benign and Scalable Synthesis of 2- and 4-substituted Benzyliperidines", European Journal of Organic Chemistry, vol. 17, 2004, pages 3623-3632, XP002346569, DOI: doi:10.1002/ejoc.200400215
- XU CHEN ET AL: "Synthesis of Heteroaryl Compounds through Cross-Coupling Reaction of Aryl Bromides or Benzyl Halides with Thienyl and Pyridyl Aluminum Reagents", JOURNAL OF ORGANIC CHEMISTRY, vol. 79, no. 1, 11 December 2013 (2013-12-11), pages 230-239, XP055753685, Japan ISSN: 0022-3263, DOI: 10.1021/jo4024123

## Description

### Technical Field

The present invention relates to a method of stably storing and supplying hydrogen through hydrogenation and dehydrogenation chemical reactions using 2-(methylbenzyl)pyridine as hydrogen storage material.

### Background Art

Demand for permanent and environmentally friendly renewable energy is explosively increasing because of environmental pollution problems such as exhaustion of fossil fuels and global warming. However, it is difficult to balance the supply and demand of renewable energy owing to the discontinuity of energy sources depending on time and the natural environment. As the rate of generation of renewable energy increases worldwide, the demand and importance of various technologies to be used in combination with other technologies as well as renewable energy are gradually increasing for energy efficiency improvement and stable energy supply.

In order to replace existing fossil fuels and solve the problem of instability of renewable energy generation, continuous energy supply is needed, and thus it is urgent to develop technologies for storing and supplying surplus renewable energy. A variety of storage methods for storing electric energy produced from renewable energy have been developed, but a method of using hydrogen as an energy storage material through a P2G (Power to Gas) process is emerging in order to ensure the storage capacity and long duration.

Hydrogen may be produced using solar energy or wind power, and is applicable in a wide variety of places, and thus is regarded as a future energy source not only in developed countries but also in Korea. Hydrogen has very high energy storage capacity per weight but very low energy storage capacity per volume, making it great difficult to commercialize hydrogen energy.

With the goal of solving this problem, individual countries have invested a lot of research and development costs for years. Examples of efficient hydrogen storage methods developed to date include a CGH2 method of compressing hydrogen at a pressure of 700 bar and an LH2 method of storing liquid hydrogen through liquefaction of hydrogen at -253°C. However, these methods have to be performed at low temperatures, and the density of liquid hydrogen is also low, namely 71.2 kg/m³.

Room-temperature gaseous hydrogen storage technologies using physical absorption of MOF or porous inorganic nanomaterials have problems of low energy storage density, and require large amounts of energy for the storage and desorption of hydrogen, making it difficult to realize miniaturization and modularization thereof.

There is a chemical hydrogen storage technology through a reversible catalyst (de)hydrogenation reaction using a liquid organic hydrogen carrier (LOHCs). LOHC technology is capable of storing 5 wt% or more of hydrogen, as recommended by the U.S. Department of Energy (DOE), and has the advantages of ease of technology implementation due to the low pressure operating range and easy recycling of storage materials. Furthermore, LOHC technology is also becoming an energy storage process that enables efficient transport and storage using existing liquid fuel transport and storage infrastructures due to the manageable physical properties and high energy density of liquid fossil fuels.

As LOHC materials, cyclic compounds such as benzene, toluene, naphthalene, and biphenyl compounds were initially studied, but are volatile and are thus limited in usefulness. In recent years, thiophene, quinaldine and carbazole have been developed as LOHC materials.

As a conventional hydrogen storage material, U.S. Patent No. 7,351,395 (Patent Document 1) discloses reversible hydrogen occlusion using a compound in the form of a pi-conjugated substrate, but the use of a mixture is required due to the difficulty of obtaining uniform reactivity through the pi-conjugation and to the fact that the phase thereof is mostly solid. In particular, when using N-ethylcarbazole (NEC)/hydrogenated N-ethylcarbazole (H12-NEC), the theoretical hydrogen storage capacity thereof is 5.8 wt%, and thorough research into the rates of hydrogenation and dehydrogenation has been carried out, but limitations are imposed on transport and storage owing to the existence in a solid phase at room temperature because of the high melting point (68°C) of NEC.

With the goal of overcoming the limitations of NEC, Korean Patent Application Publication No. 10-2015-0097558 (Patent Document 2) discloses the use of benzyltoluene and dibenzyltoluene as hydrogen storage materials. These materials are liquids at room temperature and have a boiling point of 280°C or higher and thus are not limited in use but are limited in hydrogenation and dehydrogenation reactions by the restriction of the hydrocarbon structure. In particular, the rate of dehydrogenation thereof is low compared to NEC, and hence dehydrogenation has to be carried out at a high temperature of at least 300°C.

Also, Chinese Patent Application Publication No. 20151-0005811 (Patent Document 3) discloses the use of unsaturated aromatic hydrocarbons or unsaturated heterocyclic compounds, such as N-n-propyl diphenylene imine, N-ethylcarbazole, N-methylindole, and the like, as hydrogen storage materials, but still do not solve problems related to thermal stability, reactivity, etc. Therefore, the development of a novel hydrogen storage material, which is operable at low temperatures and is excellent in thermal stability, is urgently required.

DE 102014210464 A1 relates to the use of at least one substrate for storing hydrogen, wherein the at least one substrate comprises at least one aromatic hydrocarbon and at least one N-heteroaromatic hydrocarbon, wherein the at least one aromatic hydrocarbon and the at least one N-heteroaromatic hydrocarbon are linked to each other via at least one chemical bond such that the at least one aromatic hydrocarbon and the at least one N-heteroaromatic hydrocarbon are in conjugation with each other.

AGAI, B. et al.: "Convenient, Benign and Scalable Synthesis of 2- and 4-substituted Benzyliperidines", European Journal of Organic Chemistry, vol. 17, 2004, pages 3623-3632 discloses a short, scalable and environmentally benign synthesis of 2- and 4-substituted benzylpiperidines has been developed. The method is based on the temperature-programmed consecutive deoxygenation and heteroaromatic ring saturation of aryl(pyridin-2-yl)- and aryl(pyridin-4-yl)methanols and aryl(pyridin-4-yl)methanones in the presence of Pd/C catalyst. The crucial roles of the temperature, the acidity and the substrate structure in the change of selectivity have been demonstrated by isolation of several substituted aryl(piperidine)methanols. The carbinols and ketones were prepared from commercially available pyridinecarbaldehydes or 4-cyanopyridine and substituted bromobenzenes via organometallic intermediates.

### Disclosure

### Technical Problem

Accordingly, the present invention is intended to provide a method for storing and releasing hydrogen according to claim 1. Preferred embodiments are set forth in the dependent claims.

.

### Technical Solution

A reference example relates to a system for storing and releasing hydrogen, comprising:
i) hydrogenating a substrate having at least one conjugated bond to store hydrogen in the substrate; and
ii) dehydrogenating the hydrogenated substrate to release hydrogen therefrom,

the substrate being a pyridine-based hydrogen storage material represented by Chemical Formula 1 below. 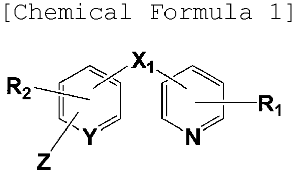
In Chemical Formula 1,
Y represents CH or N,
Z represents H or 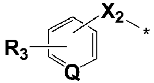 (wherein Q is CH or N),
R₁ represents H, a C₁-C₆ alkyl group, or 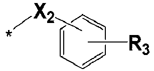 (wherein Q is CH or N),
R₂ represents H or a C₁-C₆ alkyl group,
R₃ represents H or a C₁-C₆ alkyl group, and
X₁ and X₂ each represent a single bond, -(CHR₄)ₙ-(wherein n is an integer of 1 to 3, and R₄ is H, OH, or a C₁-C₆ alkyl group), -C(=CH₂)-, -C(O)-, or -N(R₅)- (wherein R₅ is H or a C₁-C₆ alkyl group) .

The present invention provides a method of storing and releasing hydrogen according to claim 1. Preferred embodiments are set forth in the subclaims

### Advantageous Effects

Used in the system for storing and releasing hydrogen according to a reference example, a pyridine-based hydrogen storage material represented by Chemical Formula 1 is a liquid at room temperature, enables dehydrogenation at a low temperature of 280°C or less, and is thermally stable.

Also, when the pyridine-based hydrogen storage material represented by Chemical Formula 1 is subjected to dehydrogenation in the presence of a catalyst, it is superior in initial reaction rate and hydrogen supply performance compared to benzyltoluene (LH), diphenylmethane (DCM), and N-ethylcarbazole (NEC), and thus the likelihood of commercialization thereof as a material for storing and supplying hydrogen is high.

### Description of Drawings

FIG. 1 is a graph showing the dehydrogenation enthalpy and hydrogen storage capacity of a hydrogen storage material calculated using a density functional theory (DFT) calculation process;

FIG. 2 is a graph showing the results of measurement of hydrogen evolution (H₂ evolution) over time depending on the kind of catalyst in a dehydrogenation reaction of a 2-(methylbenzyl)pyridine hydrogen storage material;

FIG. 3 is a graph showing the results of comparison of initial reaction rate and hydrogen supply performance of 2-(methylbenzyl)pyridine (NLH), benzyltoluene (LH), and diphenylmethane (DCM), serving as hydrogen storage materials; and

FIG. 4 is a graph showing the results of comparison of initial reaction rate and hydrogen supply performance of 2-(methylbenzyl)pyridine (NLH) and N-ethylcarbazole (NEC), serving as hydrogen storage materials.

### Best Mode

Hereinafter, a detailed description will be given of embodiments of reference examples.

A reference example pertains to a system for storing and releasing hydrogen using a pyridine-based hydrogen storage material represented by Chemical Formula 1 below.

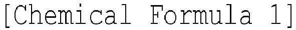

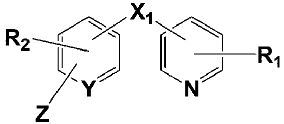

In Chemical Formula 1,
Y represents CH or N,
Z represents H or 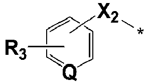 (wherein Q is CH or N),
R₁ represents H, a C₁-C₆ alkyl group, or 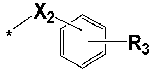 (wherein Q is CH or N),
R₂ represents H or a C₁-C₆ alkyl group,
R₃ represents H or a C₁-C₆ alkyl group, and
X₁ and X₂ each represent a single bond, -(CHR₄)ₙ-(wherein n is an integer of 1 to 3, and R₄ is H, OH, or a C₁-C₆ alkyl group), -C(=CH₂)-, -C(O)-, or -N(R₅)- (wherein R₅ is H or a C₁-C₆ alkyl group) .

The pyridine-based hydrogen storage material represented by Chemical Formula 1 has a melting point of 20°C or lower so as to maintain a liquid phase at room temperature. Specifically, the pyridine-based hydrogen storage material represented by Chemical Formula 1 has a melting point of -50°C to 20°C. Thus, the system for storing and releasing hydrogen using the pyridine-based hydrogen storage material represented by Chemical Formula 1 is advantageous in that there is no need to use an additional solvent or additive in order to improve reactivity.

Also, the pyridine-based hydrogen storage material represented by Chemical Formula 1 has a boiling point of 250°C or higher, and is thus capable of remaining stable during dehydrogenation. Specifically, the pyridine-based hydrogen storage material represented by Chemical Formula 1 has a boiling point of 250°C to 400°C.

According to a reference example, the pyridine-based hydrogen storage material may be specifically represented by Chemical Formula 1a below.

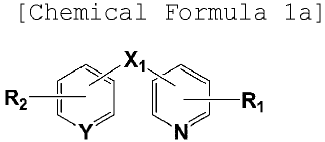

(In Chemical Formula 1a, Y represents CH or N; R₁ represents H or a C₁-C₆ alkyl group; R₂ represents H or a C₁-C₆ alkyl group; and X₁ represents -(CHR₄)ₙ- (wherein n is an integer of 1 to 3, and R₄ represents H, OH, or a C₁-C₆ alkyl group)) .

Also, the pyridine-based hydrogen storage material according to a reference example may be specifically represented by Chemical Formula 1b below.

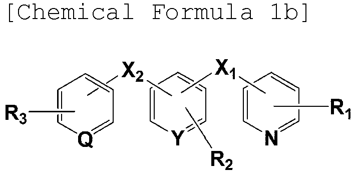

(In Chemical Formula 1b, Y represents CH or N; R₁ represents H or a C₁-C₆ alkyl group; R₂ represents H or a C₁-C₆ alkyl group; R₃ represents H or a C₁-C₆ alkyl group; and X₁ and X₂ each represent a single bond, -(CHR₄)ₙ- (wherein n is an integer of 1 to 3, and R₄ is H, OH, or a C₁-C₆ alkyl group), -C(=CH₂)-, -C(O)-, or -N(R₅)- (wherein R₅ is H or a C₁-C₆ alkyl group)).

Also, the pyridine-based hydrogen storage material according to a reference example may be specifically represented by Chemical Formula 1c below.

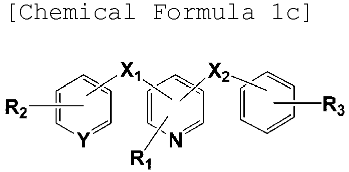

(In Chemical Formula 1c, Y represents CH or N, R₁ represents H or a C₁-C₆ alkyl group, R₂ represents H or a C₁-C₆ alkyl group, R₃ represents H or a C₁-C₆ alkyl group, and X₁ and X₂ each represent a single bond or -(CHR₄)ₙ- (wherein n is an integer of 1 to 3, and R₄ is H, OH, or a C₁-C₆ alkyl group)).

Also, specific examples of the pyridine-based hydrogen storage material according to a reference example are given in Table 1 below.

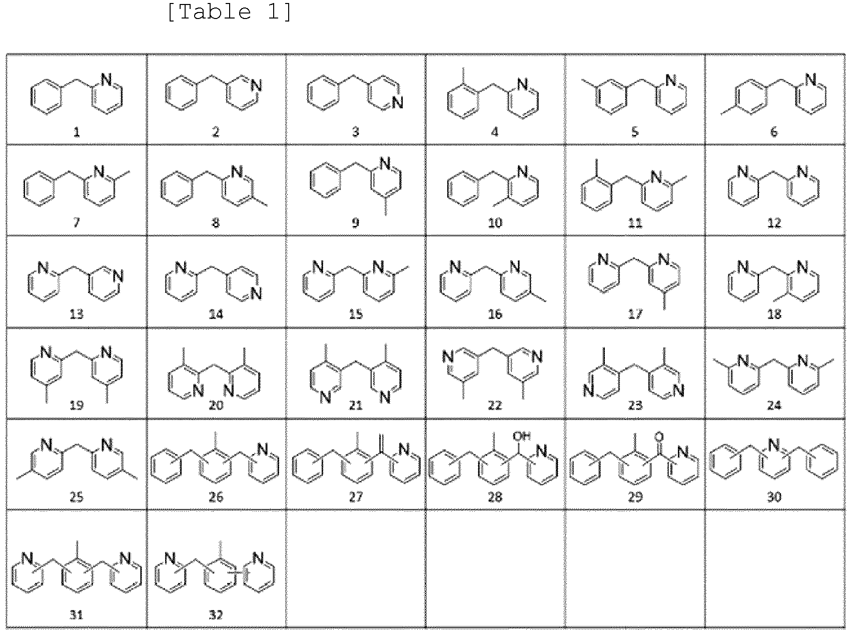

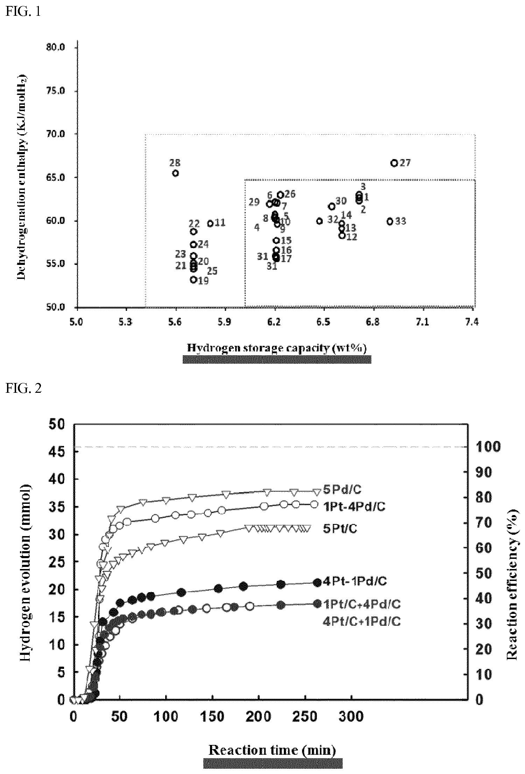

In order to confirm the usability of the pyridine-based hydrogen storage material proposed in the present invention, the dehydrogenation enthalpy and the hydrogen storage capacity of each of the compounds specifically illustrated in Table 1 were calculated using the density functional theory (DFT) calculation process. As shown in FIG. 1, the pyridine-based hydrogen storage material proposed in the present invention has a hydrogen storage capacity of 5 wt% or more, as recommended by the U.S. Department of Energy (DOE), and has a dehydrogenation enthalpy of 70 kJ/mol_{-H2} or less. Furthermore, some pyridine-based hydrogen storage materials have hydrogen storage capacities of 6 wt% or more, at most 7.0 wt%. Most dehydrogenation enthalpies are 65 kJ/mol_{-H2} or less, and some thereof are 55 kJ/mol_{-H2}.

Examples of the pyridine-based compound represented by Chemical Formula 1, proposed as a hydrogen storage material in the present invention, are mostly known, and some new compounds may be synthesized using a typical organic synthesis process.

Hereinafter, a detailed description will be given of embodiments of the present invention. The present invention pertains to a method of storing and releasing hydrogen according to claim 1. Preferred embodiments are set forth in the subclaims.

The method of storing and releasing hydrogen according to the present invention is capable of storing or releasing hydrogen by bringing the pyridine-based hydrogen storage material into contact with a metal catalyst in a reactor.

In a preferred embodiment of the present invention, a metal catalyst for hydrogenation or dehydrogenation is used to store or release hydrogen.

Specifically, the metal catalyst for hydrogenation or dehydrogenation may be a catalyst in which a Group VIIIB metal element in a standard periodic table is supported as an active component. The catalyst in which a Group VIIIB metal element is supported may be a catalyst in which ruthenium (Ru), platinum (Pt), palladium (Pd), nickel (Ni), iridium (Ir), rhodium (Rh) and iron (Fe), which are used alone or in combinations or alloys of two thereof, are supported.

Also, the metal catalyst may be a supported catalyst prepared by distributing a metal catalyst active component in a fine form on a support having a large specific surface area or a porous support.

The support is one that is typically used in the catalyst preparation field, and the choice thereof is not particularly limited in the present invention. Representative examples of the support may include porous materials such as activated carbon(C), alumina, silica, and zeolite. The amount of the metal catalyst active component that is supported is 0.005 to 0.1 wt%, and preferably 0.01 to 0.05 wt%, based on the weight of the support.

Specific examples of the hydrogenation catalyst may include a Ru/Al₂O₃ catalyst, a Pt/Al₂O₃ catalyst, a Pd/Al₂O₃ catalyst, a Ru-Pt/Al₂O₃ catalyst, a Ru-Pd/Al₂O₃ catalyst, a Pt-Pd/Al₂O₃ catalyst, a mixed catalyst of Pt/Al₂O₃ and Ru/Al₂O₃, and a mixed catalyst of Pt/Al₂O₃ and Pd/Al₂O₃, but the hydrogenation catalyst of the present invention is not limited thereto. Also, specific examples of the dehydrogenation catalyst may include a Ru/C catalyst, a Pt/C catalyst, a Pd/C catalyst, a Ru-Pt/C catalyst, a Ru-Pd/C catalyst, a Pt-Pd/C catalyst, and a mixed catalyst of Pt/C and Pd/C, but the dehydrogenation catalyst of the present invention is not limited thereto.

Also, hydrogen may be stored through hydrogenation, and the reaction temperature is 30°C to 250°C, preferably 80°C to 200°C, and more preferably 120°C to 180°C. The reaction pressure is 10 bar to 200 bar, preferably 20 bar to 100 bar, and more preferably 30 bar to 60 bar.

Also, hydrogen may be supplied through dehydrogenation, and the reaction temperature is 200°C to 310°C, and preferably 240°C to 270°C. The reaction pressure is atmospheric pressure.

A better understanding of the present invention is given through the following examples, which are set forth to illustrate but are not to be construed as limiting the present invention.

### [Examples]

### Preparation Example. Preparation of 2-(methylbenzyl)pyridine

### (1) Synthesis of 2-chloromethylpyridine

While a mixed solution of 250 mL of dichloromethane and 19.4 mL (202 mmol) of 2-pyridinemethanol was stirred, a mixed solution of 250 mL of dichloromethane and 21.1 mL (290 mmol) of thionyl chloride was slowly added dropwise thereto. After stirring for 16 hr, a saturated sodium bicarbonate aqueous solution was added to neutralize the solution, after which the organic layer was separated. The separated organic layer was further extracted using a saturated sodium chloride aqueous solution. The resulting organic layer was dried over magnesium sulfate and then concentrated under reduced pressure, thereby obtaining 2-chloromethylpyridine at a yield of 90%.

### (2) Synthesis of 2-(methylbenzyl)pyridine

While a mixed solution of 150 mL of toluene and 42.7 g (320 mmol) of anhydrous aluminum chloride (AlCl₃) was stirred, 2-chloromethylpyridine was added dropwise thereto. After completion of the dropwise addition, the reaction temperature was elevated to 90°C, followed by reflux with stirring for 16 hr. After termination of the reaction, stirring was performed and then a saturated sodium hydroxide aqueous solution was added to neutralize the solution, after which the organic layer was separated. The separated organic layer was further extracted using a saturated sodium chloride aqueous solution. The resulting organic layer was dried over magnesium sulfate and then concentrated under reduced pressure, after which the crude solution thus obtained was subjected to vacuum distillation at 0.01 torr and 104°C, thereby yielding 2-(methylbenzyl)pyridine as a pale yellow liquid.

The 2-(methylbenzyl)pyridine obtained in Preparation Example is present in the form of a mixture comprising 40 wt% of 2-(2-methylbenzyl)pyridine, 25 wt% of 2-(3-methylbenzyl)pyridine and 35 wt% of 2-(4-methylbenzyl)pyridine. In the present invention, the above mixture is represented as 2-(methylbenzyl)pyridine.

### Example 1. Evaluation of hydrogenation efficiency of 2-(methylbenzyl)pyridine (NLH) depending on the kind of catalyst

Hydrogenation was carried out using 2-(methylbenzyl)pyridine (NLH) as a hydrogen storage material, thus affording hydrogenated 2-((methylcyclohexyl)methyl)piperidine (H₁₂-NLH). The hydrogenation reaction was progressed using 2-(methylbenzyl)pyridine (NLH) in a high-pressure reactor at 150°C under a hydrogen pressure of 40 bar without the use of an additional solvent.

The 2-((methylcyclohexyl)methyl)piperidine prepared through hydrogenation in Example 1 is a mixture comprising 2-((2-methylcyclohexyl)methyl)piperidine, 2-((3-methylcyclohexyl)methyl)piperidine and 2-((4-methylcyclohexyl)methyl)piperidine. In the present invention, the above mixture is represented as 2-((methylcyclohexyl)methyl)piperidine.

The results of measurement of hydrogenation efficiency depending on the kind of catalyst used for the hydrogenation of 2-(methylbenzyl)pyridine are shown in Table 2 below.

**[Table 2]**

| Hydrogenation catalyst | Catalyst used (wt%) | Hydrogenation Efficiency (%) |
|---|---|---|
| 5 wt% Ru/Al₂O₃ | 0.86 % | 97.5 |
| | 1.41 % | 100 |
| 5 wt% Pt/Al₂O₃ | 1.41 % | 62.6 |
| | 12.9 % | 96.4 |
| 5 wt% Pd/Al₂O₃ | 0.86 % | 55.6 |
| | 1.41 % | 60.2 |
| | 12.9 % | 97.3 |
| * Catalyst used: the amount (g) of an active component in a supported catalyst used relative to the weight (g) of a substrate is expressed as a percentage. | | |

As is apparent from Table 2, the hydrogen storage reaction was efficiently progressed using a ruthenium (Ru) catalyst, a platinum (Pt) catalyst and a palladium (Pd) catalyst. In particular, when the hydrogenation reaction was carried out at a reaction temperature of 150°C under a hydrogen pressure of 40 bar, the ruthenium (Ru) catalyst resulted in a high hydrogenation efficiency of 97.5% within 2 hr using the active component in a small amount of 0.86 wt%.

### Example 2. Evaluation of hydrogen evolution of 2-((methylcyclohexyl)methyl)piperidine (H₁₂-NLH) depending on the kind of catalyst

The hydrogenated 2-((methylcyclohexyl)methyl)piperidine (H₁₂-NLH) obtained in Example 1 was subjected to dehydrogenation at 270°C under atmospheric pressure. In Example 2, as the catalyst for dehydrogenation, (A) a 5Pt/C catalyst, (B) a 5Pd/C catalyst, (C) a 4Pt-1Pd/C catalyst, (D) a 1Pt-4Pd/C catalyst, (E) a mixed catalyst of 4Pt/C + 1Pd/C, and (F) a mixed catalyst of 1Pt/C + 4Pd/C were used. Here, the numeral in front of each metal designates the amount (wt%) of each metal that is supported relative to the weight of the support.

FIG. 2 shows the results of measurement of hydrogen evolution over time depending on the kind of catalyst used for dehydrogenation. As shown in FIG. 2, the hydrogen evolution was decreased in the sequence of (B) the 5Pd/C catalyst, (D) the 1Pt-4Pd/C catalyst, (A) the 5Pt/C catalyst, (C) the 4Pt-1Pd/C catalyst, (F) the mixed catalyst of 1Pt/C + 4Pd/C, and (E) the mixed catalyst of 4Pt/C + 1Pd/C.

### Example 3. Evaluation of hydrogen evolution using 2-(methylbenzyl)pyridine in comparison to reference substrates in the presence of Pd/C catalyst

In Example 3, the results of hydrogen supply performance of 2-(methylbenzyl)pyridine (NLH), benzyltoluene (LH), diphenylmethane (DCM), and N-ethylcarbazole (NEC), serving as hydrogen storage materials, were compared. Specifically, 2-((methylcyclohexyl)methyl)piperidine, 1-(cyclohexylmethyl)-2-methylcyclohexane, and dicyclohexylmethane were subjected to dehydrogenation at 270°C under atmospheric pressure in the presence of a Pd/C catalyst, thus preparing 2-(methylbenzyl)pyridine (NLH), benzyltoluene (LH), diphenylmethane (DCM), and N-ethylcarbazole (NEC).

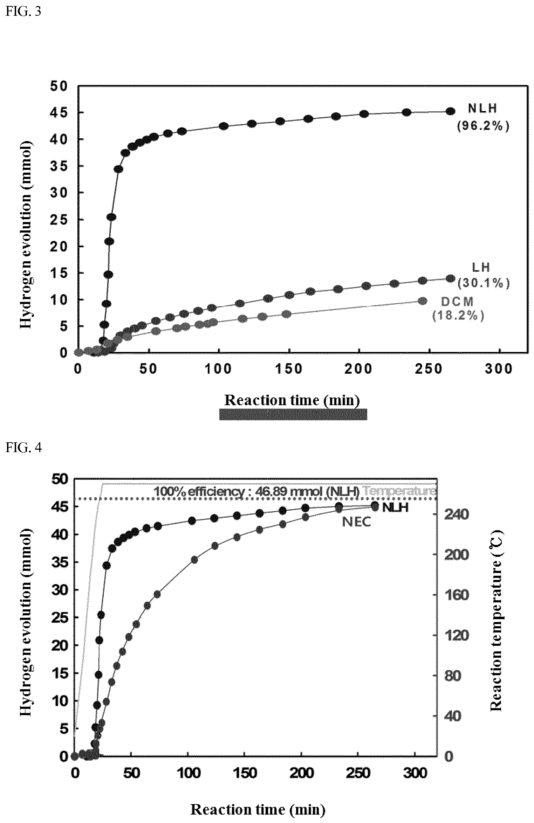

The graph of FIG. 3 shows the results of comparison of initial reaction rate and hydrogen supply performance of 2-(methylbenzyl)pyridine (NLH), benzyltoluene (LH), and diphenylmethane (DCM). As shown in FIG. 3, 2-(methylbenzyl)pyridine (NLH) can be confirmed to exhibit a very high initial reaction rate and very high hydrogen supply performance compared to benzyltoluene (LH) and diphenylmethane (DCM).

Also, the graph of FIG. 4 shows the results of comparison of initial reaction rate and hydrogen supply performance of hydrogenation compounds of 2-(methylbenzyl)pyridine (NLH) and N-ethylcarbazole (NEC). As shown in FIG. 4, 2-(methylbenzyl)pyridine (NLH) can be confirmed to exhibit a very high initial reaction rate and very high hydrogen supply performance compared to N-ethylcarbazole (NEC). Moreover, N-ethylcarbazole (NEC), having a melting point of 68°C, is present as a solid at room temperature and is thus disadvantageous in that it essentially requires a solvent in the construction of a system for storing and releasing hydrogen, whereas 2-(methylbenzyl)pyridine (NLH) is a liquid at room temperature and is thus advantageous in that it obviates the use of a solvent.

### Industrial Applicability

According to the present invention, the inventive method for storing and releasing hydrogen enables dehydrogenation at a low temperature of 280°C or less, 2-(methylbenzyl)pyridine is thermally stable, and is superior in initial reaction rate and hydrogen supply performance compared to benzyltoluene (LH), diphenylmethane (DCM), and N-ethylcarbazole (NEC), and is thus industrially applicable.

## Claims

1. A method of storing and releasing hydrogen, comprising:
a) hydrogenating a substrate having at least one conjugated bond in presence of a hydrogenation catalyst to store hydrogen in the substrate; and
b) dehydrogenating the hydrogenated substrate in presence of a dehydrogenation catalyst to release hydrogen therefrom,
the substrate being a pyridine-based hydrogen storage material is 2-(methylbenzyl)pyridine.

2. The method of claim 1, wherein the hydrogenation catalyst used in step a) is a catalyst in which a Group VIIIB metal element in a standard periodic table is supported as an active component.

3. The method of claim 1, wherein the hydrogenation catalyst used in step a) is a catalyst in which any one or two selected from among ruthenium (Ru), platinum (Pt) and palladium (Pd) are supported as an active component.

4. The method of claim 1, wherein the hydrogenation catalyst used in step a) is a Ru/Al₂O₃ catalyst, a Pt/Al₂O₃ catalyst, a Pd/Al₂O₃ catalyst, a Ru-Pt/Al₂O₃ catalyst, a Ru-Pd/Al₂O₃ catalyst, a Pt-Pd/Al₂O₃ catalyst, or a mixture thereof.

5. The method of claim 1, wherein the dehydrogenation catalyst used in step b) is a catalyst in which a Group VIIIB metal element in a standard periodic table is supported as an active component.

6. The method of claim 1, wherein the dehydrogenation catalyst used in step b) is a catalyst in which any one or two selected from among ruthenium (Ru), platinum (Pt) and palladium (Pd) are supported as an active component.

7. The method of claim 1, wherein the dehydrogenation catalyst used in step b) is a Ru/C catalyst, a Pt/C catalyst, a Pd/C catalyst, a Ru-Pt/C catalyst, a Ru-Pd/C catalyst, a Pt-Pd/C catalyst, or a mixture thereof.

8. The method of claim 1, wherein the hydrogenating in step a) is carried out at a reaction temperature of 30°C to 250°C under a pressure of 10 bar to 200 bar.

9. The method of claim 1, wherein the dehydrogenating in step b) is carried out at a reaction temperature of 200°C to 310°C under atmospheric pressure.

## Patentansprüche

1. Verfahren zur Speicherung und Freisetzung von Wasserstoff, umfassend:
a) Hydrieren eines Substrats mit mindestens einer konjugierten Bindung in Gegenwart eines Hydrierungskatalysators, um Wasserstoff in dem Substrat zu speichern; und
b) Dehydrieren des hydrierten Substrats in Gegenwart eines Dehydrierungskatalysators, um daraus Wasserstoff freizusetzen,
wobei es sich bei dem Substrat, das ein Wasserstoffspeichermaterial auf der Basis von Pyridin ist, um 2-(Methylbenzyl)-Pyridin handelt.

2. Verfahren nach Anspruch 1, wobei der in Schritt a) verwendete Hydrierungskatalysator ein Katalysator ist, bei dem ein Metallelement der Gruppe VIIIB im Standardperiodensystem als aktive Komponente unterstützt wird.

3. Verfahren nach Anspruch 1, wobei der in Schritt a) verwendete Hydrierungskatalysator ein Katalysator ist, bei dem ein oder zwei aus Ruthenium (Ru), Platin (Pt) und Palladium (Pd) ausgewählte Elemente als aktive Komponente unterstützt werden.

4. Verfahren nach Anspruch 1, wobei der in Schritt a) verwendete Hydrierungskatalysator ein Ru/Al₂O₃-Katalysator, ein Pt/Al₂O₃-Katalysator, ein Pd/Al₂O₃-Katalysator, ein Ru-Pt/Al₂O₃-Katalysator, ein Ru-Pd/Al₂O₃-Katalysator, ein Pt-Pd/Al₂O₃-Katalysator oder eine Mischung davon ist.

5. Verfahren nach Anspruch 1, wobei der in Schritt b) verwendete Dehydrierungskatalysator ein Katalysator ist, bei dem ein Metallelement der Gruppe VIIIB im Standardperiodensystem als aktive Komponente unterstützt wird.

6. Verfahren nach Anspruch 1, wobei der in Schritt b) verwendete Dehydrierungskatalysator ein Katalysator ist, bei dem ein oder zwei aus Ruthenium (Ru), Platin (Pt) und Palladium (Pd) ausgewählte Elemente als aktive Komponente unterstützt werden.

7. Verfahren nach Anspruch 1, wobei der in Schritt b) verwendete Dehydrierungskatalysator ein Ru/C-Katalysator, ein Pt/C-Katalysator, ein Pd/C-Katalysator, ein Ru-Pt/C-Katalysator, ein Ru-Pd/C-Katalysator, ein Pt-Pd/C-Katalysator oder eine Mischung davon ist.

8. Verfahren nach Anspruch 1, wobei die Hydrierung in Schritt a) bei einer Reaktionstemperatur von 30°C bis 250°C unter einem Druck von 10 bar bis 200 bar ausgeführt wird.

9. Verfahren nach Anspruch 1, wobei die Dehydrierung in Schritt b) bei einer Reaktionstemperatur von 200°C bis 310°C unter atmosphärischem Druck ausgeführt wird.

## Revendications

1. Procédé de stockage et de libération d'hydrogène, comprenant :
a) hydrogéner un substrat ayant au moins une liaison conjuguée en présence d'un catalyseur d'hydrogénation pour stocker de l'hydrogène dans le substrat ; et
b) déshydrogéner le substrat hydrogéné en présence d'un catalyseur de déshydrogénation pour en libérer l'hydrogène,
dans lequel le substrat, qui est un matériau de stockage d'hydrogène à base de pyridine, est la 2-(méthylbenzyl)-pyridine.

2. Procédé selon la revendication 1, dans lequel le catalyseur d'hydrogénation utilisé dans l'étape a) est un catalyseur dans lequel un élément métallique du groupe VIIIB dans le tableau périodique standard est soutenu en tant qu'un composant actif.

3. Procédé selon la revendication 1, dans lequel le catalyseur d'hydrogénation utilisé dans l'étape a) est un catalyseur dans lequel un ou deux éléments choisis parmi le ruthénium (Ru), le platine (Pt) et le palladium (Pd) sont soutenus en tant qu'un composant actif.

4. Procédé selon la revendication 1, dans lequel le catalyseur d'hydrogénation utilisé dans l'étape a) est un catalyseur Ru/Al₂O₃, un catalyseur Pt/Al2O₃, un catalyseur Pd/Al₂O₃, un catalyseur Ru-Pt/Al₂O₃, un catalyseur Ru-Pd/Al₂O₃, un catalyseur Pt-Pd/Al₂O₃ ou un mélange de ces catalyseurs.

5. Procédé selon la revendication 1, dans lequel le catalyseur de déshydrogénation utilisé dans l'étape b) est un catalyseur dans lequel un élément métallique du groupe VIIIB dans le tableau périodique standard est soutenu en tant qu'un composant actif.

6. Procédé selon la revendication 1, dans lequel le catalyseur de déshydrogénation utilisé dans l'étape b) est un catalyseur dans lequel un ou deux éléments choisis parmi le ruthénium (Ru), le platine (Pt) et le palladium (Pd) sont soutenus en tant qu'un composant actif.

7. Procédé selon la revendication 1, dans lequel le catalyseur de déshydrogénation utilisé dans l'étape b) est un catalyseur Ru/C, un catalyseur Pt/C, un catalyseur Pd/C, un catalyseur Ru-Pt/C, un catalyseur Ru-Pd/C, un catalyseur Pt-Pd/C ou un mélange de ces catalyseurs.

8. Procédé selon la revendication 1, dans lequel l'hydrogénation dans l'étape a) est effectuée à une température de réaction de 30°C à 250°C sous une pression de 10 bars à 200 bar.

9. Procédé selon la revendication 1, dans lequel la déshydrogénation dans l'étape b) est effectuée à une température de réaction de 200°C à 310°C sous pression atmosphérique.
